# EUROPEAN PATENT SPECIFICATION

(11) **EP 1 800 232 B1**
(45) Date of publication and mention of the grant of the patent: **11.03.2009**
(21) Application number: 05796201.1
(22) Date of filing: 13.10.2005
(51) Int. Cl.: G06F 19/00, C12Q 1/68

(54) **IDENTIFICATION AND ASSIGNMENT OF FUNCTIONALLY CORRESPONDING REGULATORY SEQUENCES FOR ORTHOLOGOUS LOCI IN EUKARYOTIC GENOMES**
IDENTIFIKATION UND ZUWEISUNG VON FUNKTIONAL KORRESPONDIERENDEN REGELNDEN SEQUENZEN FÜR ORTHOLOGE ORTE IN EUKARYOTISCHEN GENOMEN
IDENTIFICATION ET ASSIGNATION DE SEQUENCES REGULATRICES CORRESPONDANTES SUR LE PLAN FONCTIONNEL POUR DES LOCI ORTHOLOGUES DE GENOMES EUCARYOTES

(30) Priority: 15.10.2004 US 964812
(43) Date of publication of application: 27.06.2007
(73) Proprietor: Genomatix Software GMBH, 80339 München (DE)
(72) Inventor: KLINGENHOFF, Andreas, 80935 München (DE)
(74) Representative: Weiss, Wolfgang
(86) International application number: PCT/EP2005/011029
(87) International publication number: WO 2006/040161

(56) References cited:
- HER GUOR MOUR ET AL: "Functional conserved elements mediate intestinal type fatty acid binding protein (I-FABP) expression in the gut epithelia of zebrafish larvae" DEVELOPMENTAL DYNAMICS, vol. 230, no. 4, August 2004 (2004-08), pages 734-742, XP009062507 ISSN: 1058-8388
- LEVY SAMUEL ET AL: "Enrichment of regulatory signals in conserved non-coding genomic sequence" BIOINFORMATICS (OXFORD), vol. 17, no. 10, October 2001 (2001-10), pages 871-877, XP002369628 ISSN: 1367-4803
- LOOTS GABRIELA G ET AL: "rVISTA for comparative sequence-based discovery of functional transcription factor binding sites" GENOME RESEARCH, vol. 12, no. 5, May 2002 (2002-05), pages 832-839, XP002369629 ISSN: 1088-9051
- ONYANGO P ET AL: "Sequence and comparative analysis of the mouse 1-megabase region orthologous to the human 11p15 imprinted domain." GENOME RESEARCH. NOV 2000, vol. 10, no. 11, November 2000 (2000-11), pages 1697-1710, XP002369631 ISSN: 1088-9051
- JEGGA ANIL G ET AL: "CisMols Analyzer: identification of compositionally similar cis-element clusters in ortholog conserved regions of coordinately expressed genes." NUCLEIC ACIDS RESEARCH. 1 JUL 2005, vol. 33, no. Web Server issue, 1 July 2005 (2005-07-01), pages W408-W411, XP002369632 ISSN: 1362-4962
- CARTHARIUS K ET AL: "Matlnspector and beyond: promoter analysis based on transcription factor binding sites" BIOINFORMATICS (OXFORD), vol. 21, no. 13, July 2005 (2005-07), pages 2933-2942, XP002369633 ISSN: 1367-4803
- KLINGENHOFF A ET AL: "Regulatory modules shared within gene classes as well as across gene classes can be detected by the same in silico approach" IN SILICO BIOLOGY, vol. 2, no. 1, 2002, pages S17-S26, XP009062457 ISSN: 1386-6338

## Description

The present invention relates to a method and computer program product for identifying and/or defining the regulatory sequence of a transcript within the genome of a eukaroytic organism and/or for identifying groups of functionally corresponding regulatory sequences of orthologous transcripts in different eukaryotic organisms.

### Background of the Invention

Today genomic sequence data from an increasing number of different organisms becomes available (Waterston et al., 2002; Lander et al., 2001). The quality of the data varies from short sequence fragments of several thousand base pairs from WGS (whole genome shotgun) projects to contiguous assembled sequences of whole chromosomes.

The annotation of the genomic sequences (e.g. location of genes, promoters, genomic repeats etc.) covers a similar broad range of quality and quantity. In a first step genomic sequences are usually analyzed by in silico methods to predict exon/intron structures (gene predictor) and repetitive sequence patterns. Short expressed sequences (EST) are then used to build supporting evidence for those gene predictions.

However, gene predictors are afflicted with high uncertainty - especially in case of gene start predictions. In addition, ESTs are usually only a few hundred base pairs in length and do not cover the 5' end of a transcript. Consequently, the correct prediction of the gene start is still a crucial challenge today.

Since promoters are defined as the sequences upstream of a transcriptional start site (TSS) their annotation depends crucially on the correct annotation of the corresponding gene start.

The only way for high quality annotation of gene starts is via 5' full-length cDNAs (Suzuki et al., 2001). Full-length cDNAs not only cover the coding sequence (CDS) but also contain the untranslated regions (UTR) located 5' and 3' of the CDS. Ideally they start with a real transcriptional start side (TSS) and end at the polyA tail. Only mapping of these full-length cDNAs to the genomic sequence results in a reliable annotation of existing transcripts for a gene locus. Therefore it is the only way to annotate promoter regions precisely. However, so far only for a few genome projects limited collections of full-length cDNAs are available (Ota et al., 2004; Imanishi et al., 2004; Okazaki et al ., 2002). laots et al., Genome Res., 12, 832-39, 2002 disclose a method for the identification of transcriptional regulatory sequences. One way to overcome the significant problems in the quality of 5' complete gene annotation is comparison of annotations available for orthologous loci (i.e. from different organisms). Unfortunately the divergence of the genomic sequences of even closely related organisms (e.g. rat and mouse) does not allow a genome wide mapping of full-length cDNAs from one organism to the genome of the other. Especially the non-coding UTRs of the cDNAs are only sparsely conserved between the organisms. Lowering the similarity thresholds for the mapping of these sequence regions often results in ambiguous results for the leading exon of a transcript. Accordingly, the results cannot be used for the precise annotation of promoter regions.

Simply scanning windows of sequences as a mode of length restriction does not work. The restriction by cross-species exon mapping is not obvious even to experts in the field and on top of that requires application of the precise rules outlined above to yield quality results. Due to short exon length and relatively low sequence conservation the transfer of evidence between specie is also not trivial.

The approach described here allows to evaluate existing annotation and to transfer high quality annotation from one organism to another where this information is incomplete or missing.

### Summary of the Invention

The invention relates to a method for identifying functionally corresponding regulatory sequences of orthologous transcripts within the genome of eukaroytic organisms which allows to evaluate existing annotation and to transfer high quality annotation from one organism to another where this information is incomplete or missing comprising:
(a) mapping the sequences of a plurality of orthologous transcripts within the genomes of eukaryotic organisms,
(b) identifying at least one sequence which is conserved between the orthologous transcripts of (a), wherein the sequence is a conserved exon,
(c) defining a target region for a conserved sequence in the genome of the respective eukaryotic organism based on the location of a conserved sequence identified in step (b), wherein step (c) comprises selecting a conserved sequence as an anchor for defining the target region,
(d) identifying and/or defining a regulatory sequence within the target region, wherein step (d) comprises generating a pseudotranscript sequence for the target locus, and
e) optionally assigning the regulatory sequences to groups of functionally corresponding regulatory sequences.

Further, the invention relates to a computer program product for identifying functionally corresponding regulatory sequences within the genome of eukaryotic organisms which allows to evaluate existing annotation and to transfer high quality annotation from one organism to another where this information is incomplete or missing comprising:
(a) means for mapping the sequences of a plurality of orthologous transcripts within the genomes of a plurality of eukaroytic organisms,
(b) means for identifying at least one sequence which is conserved between the orthologous transcripts of (a), wherein the sequence is a conserved exon,
(c) means for defining a target region for a conserved sequence in the genome of the respective eukaryotic organism based on the location of a conserved sequence identified by means (b), wherein means (c) comprises selecting a conserved sequence as an anchor for defining the target region,
(d) means for identifying and/or defining a regulatory sequence within the target region, wherein means (d) comprises generating a pseudotranscript sequence for the target locus, and
(e) optionally means for assigning the regulatory sequences to groups of functionally corresponding regulatory sequences.

The present invention allows the identification of groups of functionally corresponding regulatory sequences of orthologous transcripts within the genome of eukaryotic organisms. Further, the present invention allows the identification of previously unknown regulatory sequences for transcripts.

### Detailed Description of the Preferred Embodiments

### General Principle

The present method and computer program product compares and analyses the transcripts annotated for a set of orthologous loci from a group of eukaryotic organisms. Functionally corresponding regulatory sequences, which are preferably located 5' to the transcript are identified and/or characterized and assigned into groups. The regulatory sequences are preferably selected from promoters, enhancers and/or repressor regions, more preferably promoter regions. The transcripts may comprise protein coding sequences. The transcripts, however, may be or comprise functional RNA molecules.

The identification of functionally corresponding regulatory sequences is achieved by checking the orthologous transcripts for a conserved exon/intron structure. If the annotation in any of the orthologous loci is 5'-incomplete, this can now be extended by a potential conserved promoter region (termed CompGen promoter). This is carried out by mapping an exon, preferably the first exon of a transcript from one organism to the corresponding orthologous genomic sequence of the target organism.

For this purpose, the potential target region in the genomic sequence is restricted to a predetermined length of e.g. several thousand base pairs by the prior analysis of the exon/intron structure of the transcript used for the mapping. Due to this restriction of the target region it is now possible to lower the similarity thresholds to a level that is required for cross species mapping of less conserved UTRs without obtaining ambiguous results.

### Generation of homology groups with orthologous loci

In a first step, orthologous loci are identified by an exhaustive pairwise comparison of mRNA sequences available for the selected organisms. The orthologous loci are defined by matching transcripts from two or more eukaryotic organisms, e.g. a transcript from one or several organisms for which the regulatory region shall be identified and transcripts from one or several second organisms for which the regulatory region is known. Preferably, two loci are marked as orthologous if the related transcripts are pairwise best matches. A potential source for this data is the HomoloGene database provided by the National Center for Biotechnology Information (NCBI).

Based on these pairwise relations the loci are assigned to closed groups (homology groups). Two loci are connected in a homology group if they are both assigned to a common third locus but are not necessarily connected by a direct relationship. Each locus can only be member of one homology group.

The eukaroytic organisms analyzed preferably belong to the same kingdom of eukaryotic organisms, e.g. animals, plants or fungi. More preferably, the eukaryotic organisms belong to the same order, e.g. they are mammals, birds, reptils, fish, insects, etc. In general, a close relationship between the first and the second organism is preferred.

### Analyzing transcripts of the loci in a homology group

All available exon/intron-annotation for alternative transcripts for the loci in a homology group is collected from the genome annotation. Alternative transcripts differ in their exon/intron structure but may also start from different transcriptional start sites and consequently have different regulatory regions, e.g. promoter regions. Preferably, the exons of all transcripts are analyzed for conservation, i.e. the presence of conserved sequences. Two exons are considered conserved if they have an identical length, and show a sufficient sequence similarity (< 10% gaps). The sequence similarity is preferably determined using a Smith-Waterman alignment (Smith & Waterman, 1981). Preferably, the most 5' located conserved exons are used to arrange the transcripts from different loci (i.e. from different organisms) on a common scale. They represent the anchor for further distance calculations. It is noted that these exons are not necessarily the first exons, which is a major difference to 5'-complete EST assembly algorithms.

### Vertical transfer of annotation between the loci in a homology group

The first exon of each annotated transcript is mapped to the genomic sequences of all other orthologous loci (targets) in the homology group. Preferably, this is done exhaustively for all of the loci.

The mapping is preferably carried out by aligning the exon sequence and the genomic sequence (Needleman & Wunsch, 1970). To allow high quality mapping across species the potential target region for mapping is restricted. The distance between the anchor point and the transcriptional start site (TSS) of a source transcript, i.e. a transcript for which at least the TSS and preferably the regulatory region to be identified is known, is used to determine a potential location, i.e. a target or mapping region for the alignment on the genomic target sequence which is extended by preferably about 20,000 bp and more preferably up to about 10,000 bp upstream and downstream to cover the variability of the exon/intron structure of different loci. In the case of extremely large distances between the anchor point and the first exon of the source transcript (>100,000bp) the length of the target or mapping region of the genomic sequence is extended relatively to that distance (preferably up to about 20% of the distance).

For each mapping that fulfills at least one of the following criteria a pseudo transcript consisting of a single exon is generated for the target locus.
(i) The alignment preferably contains ≥70% identical nucleotides, ≤20% gaps and has ≥20bp in length.
(ii) The score calculated for the alignment normalized by the length of the alignment preferably exceeds a value of 2.

The extension and the position of the exon are derived from the mapping results. Thus the annotation for a locus is temporarily extended by conserved first exons from orthologous loci indicating a potential conserved regulatory, e.g. promoter region.

### Identification of corresponding regulatory regions

In the next step the sequences of all first exons from the different loci in a homology group are aligned against each other. These first exons may now be derived either from the genome annotation or from the pseudo transcripts generated by the mapping process described above. Suitable alignments are selected by the following criteria:
(i) The alignment contains ≥70% identical nucleotides, ≤20% gaps and has ≥50bop in length.
(ii) The alignment contains ≥60% identical nucleotides, ≤5% gaps and has ≥50bp in length.
(iii) The alignment contains ≤25% gaps and the score calculated for the alignment exceeds a value of 300.
(iv) The alignment has a length of ≥60bp and the score calculated for the alignment normalized by the length of the alignment has a value of at least 2.
   For transcripts starting with a first exon shorter than 60bp two additional criteria are used.
(v) The alignment contains ≥90% identical nucleotides, ≤20% gaps and has ≥20bp in length.
(vii) The alignment contains ≥75% identical nucleotides, ≤10% gaps and has ≥20bp in length.

For each alignment that fulfills at least one of the criteria the two corresponding transcripts are assigned as pair-wise corresponding partners. The list of pair-wise assignments is then used to build closed groups of related transcripts.

For those groups that contain more than one transcript for a locus a regulatory region, e.g. a promoter region may be calculated that covers all of the potential sites, e.g. transcriptional start sites reflecting the known variability of transcriptional initiation processes (Suzuki et al., 2001). For loci where only a pseudo transcript is assigned to a group of related transcripts a new potential promoter region (CompGen promoter) supported by annotation from orthologous loci is added to the annotation. There is no transcript assigned to these promoters, as the detailed exon/intron structure is not determined by the present method.

The regulatory regions, e.g. promoter regions may then be assigned to a group or set comprising functionally corresponding regulatory regions, i.e. regulatory regions to which a common or at least similar biological function may be assigned.

It should be noted that several groups or sets of regulatory sequences may be assigned to a single locus, particularly if several pair-wise matching partners are identified for the sequence to be analyzed.

Further, the present invention shall be explained in more detail by the following Example.

### Example

The present method was applied to the genomes of different groups of eukaryotic organisms. The first group contains the three vertebrates Homo sapiens, Mus musculus, and Rattus norvegicus. The second group comprises the genomes of the two insects Drosophila melanogaster and Anopheles gambiae.

| | homology groups | promoter sets | CompGen promoter |
|---|---|---|---|
| vertebrates | 17069 | 27253 | 26197 |
| insects | 496 | 239 | 136 |

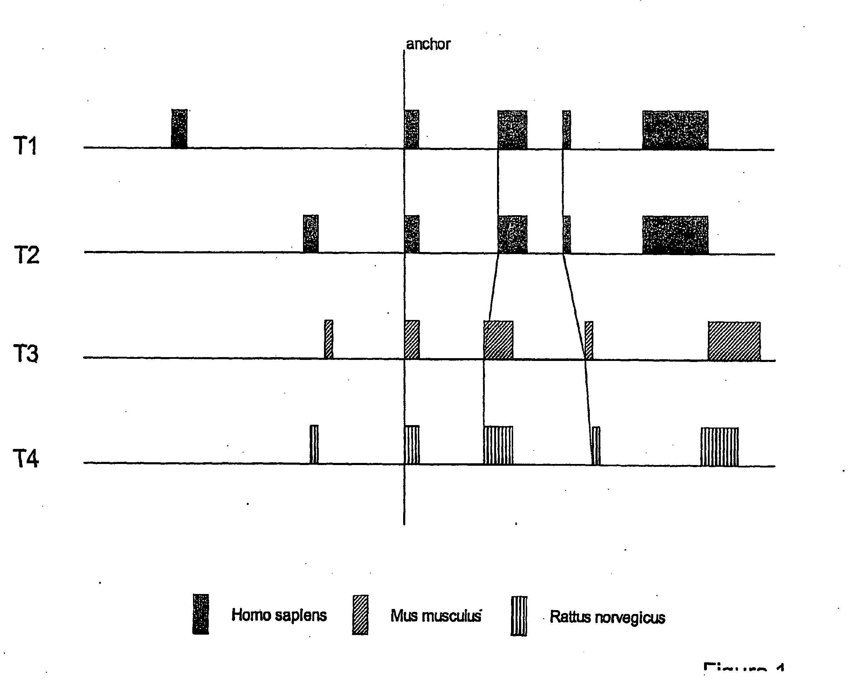

The example in Figure 1 contains four transcripts (two alternative transcripts from H.sapiens (T1 and T2), one from M.musculus (T3), and one from R.norvegicus (T4)). The exons conserved between different loci are connected by dotted lines. Exon 2 is the most 5' located conserved exon and is therefore selected as common anchor.

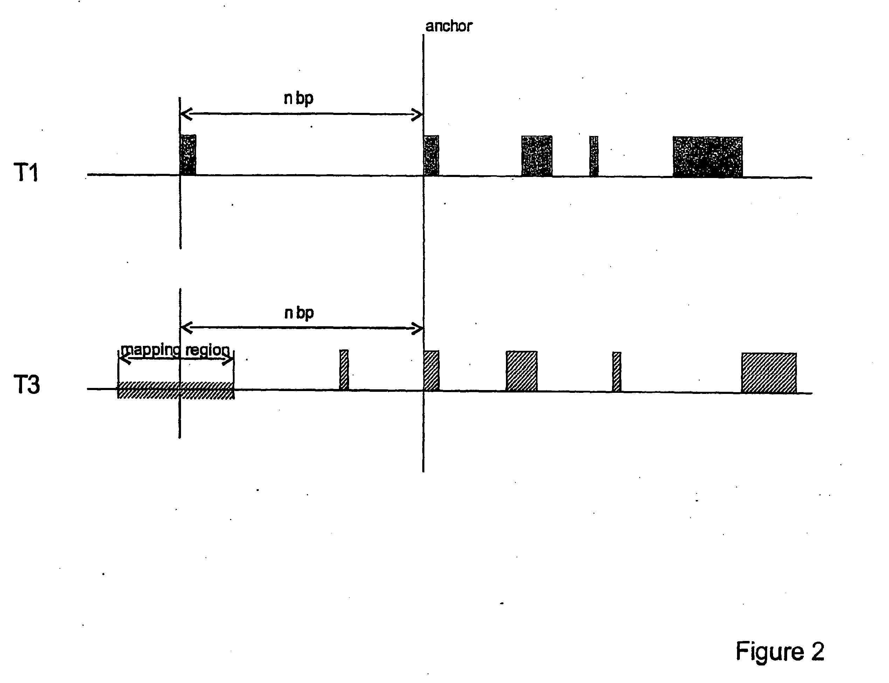

Figure 2 shows the definition of a target (mapping) region in the genomic sequence of M. musculus (T3) based on the distance (nbp) between the anchor and the TSS in the genomic sequence of H. sapiens (T1).

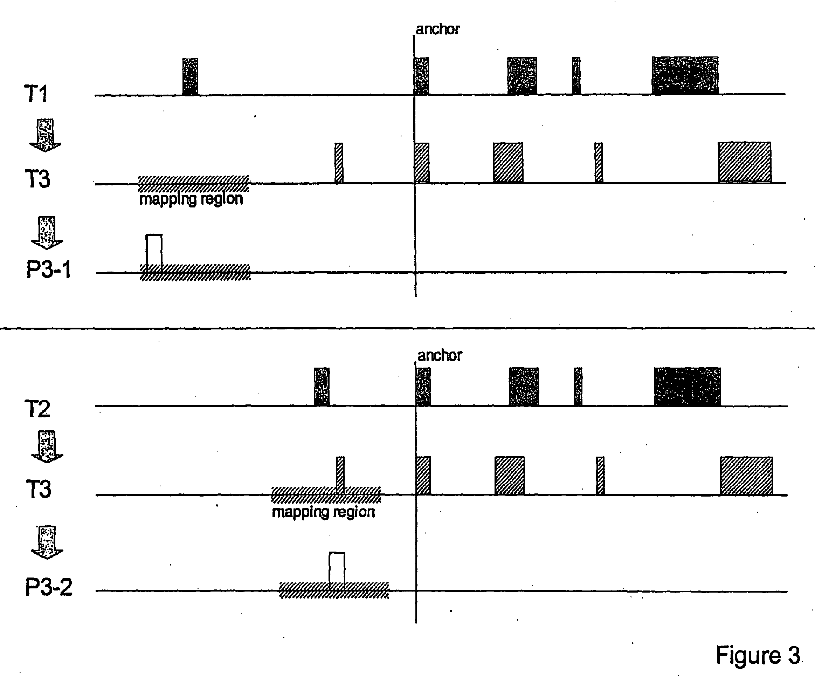

Figure 3 shows the results for the mapping of transcript T1 and T2 (human) to the genomic sequence of the murine locus (T3). The exhaustive mapping of the transcripts included in Figure 1 results in 8 pseudo transcripts (P1-3, P1-4 for H.sapiens, P3-1, P3-2, P3-4 for M.musculus and P4-1, P4-2, P4-3 for R.norvegicus).

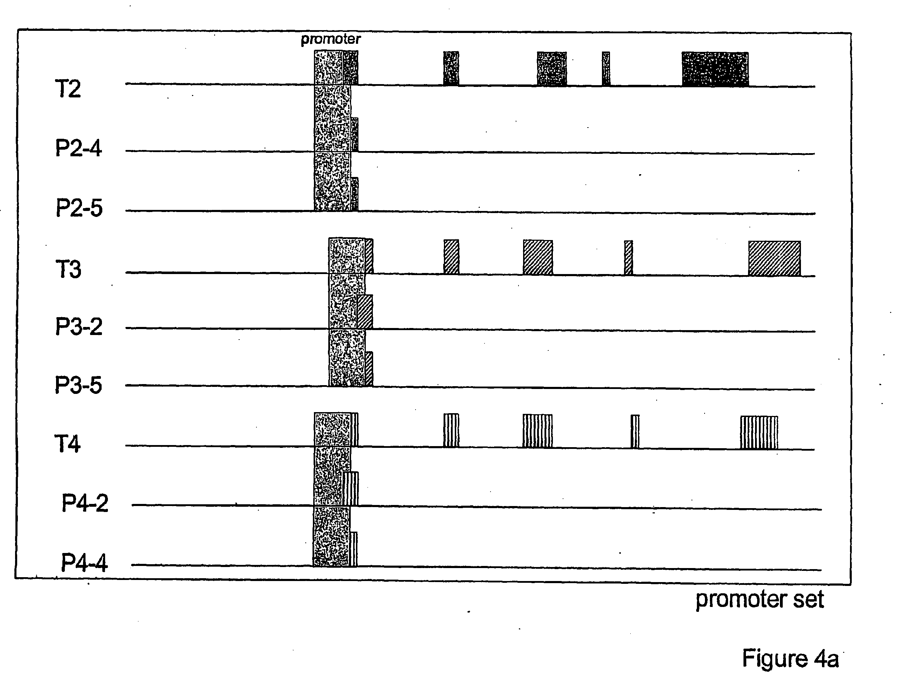

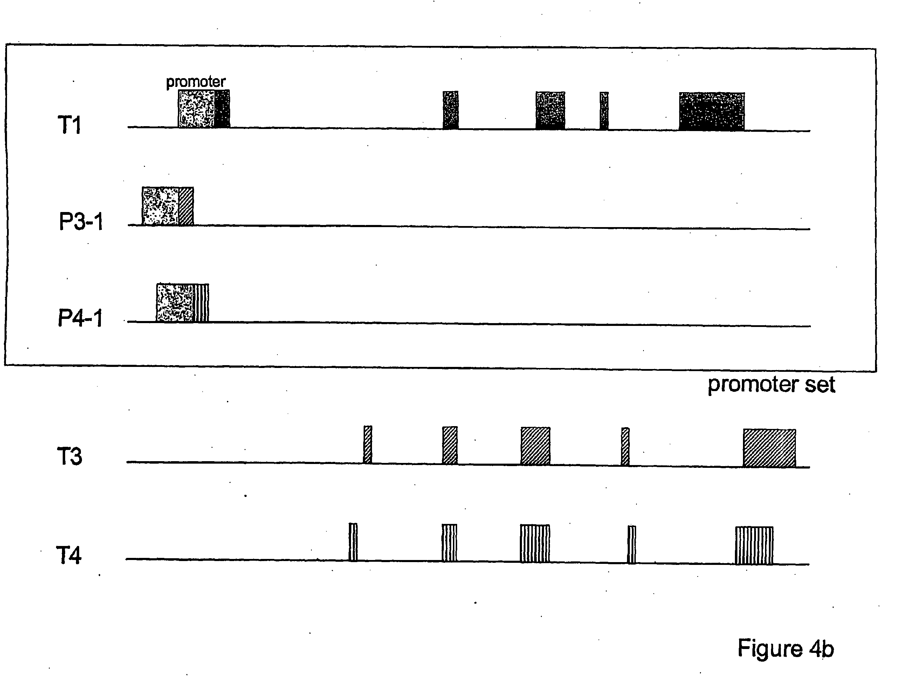

Fig. 4a and 4b show the building of closed groups of transcripts based on the sequences T1, T2, T3 and T4. In Figure 4a the calculation of promoter regions is shown for homology groups which contain more than one transcript (P2-4 and P2-5; P3-2 and P3-5; or P4-2 and P4-4) for a locus. In Figure 4b the mapping of promoter regions is shown for which only a pseudotranscript has been assigned to a homology group of related transcripts.

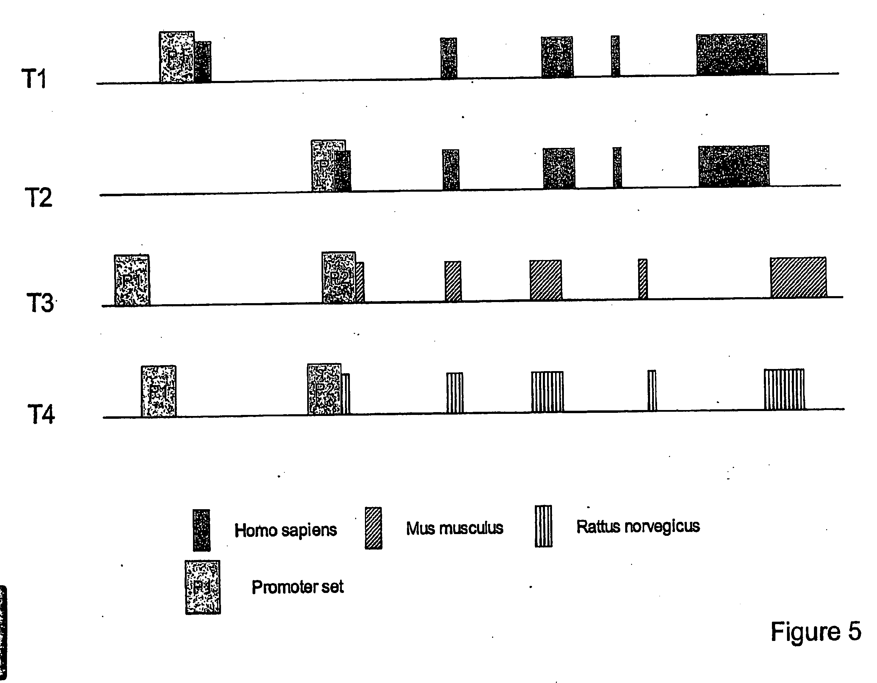

The promoter regions for M.musculus (T3) and R.norvegicus (T4) belonging to promoter set 1(P1) in Figure 5 therefore are supported by the annotation available from the human genome (T1) and by the sequence similarity detected in the two target sequences.

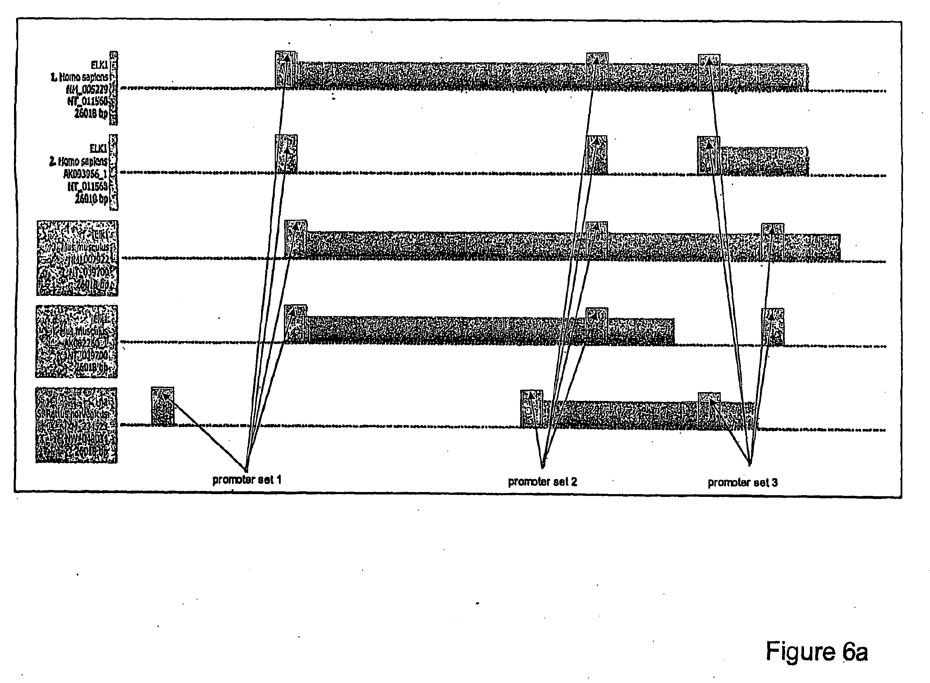

Figure 6a shows a graphical representation of the results generated by the present method for the orthologous ELK1 transcripts from Homo sapiens, Mus musculus, and Rattus norvegicus. In this example the promoters for the first human transcript (1) and the two murine transcripts (3,4) are assigned to one group (promoter set one) because of the sequence similarity of the first exon of each of the transcripts. The promoter set also contains a promoter sequence from the rat genome for which no transcript is known so far. The location of this promoter sequence was determined by the mapping of the first exons of the corresponding transcripts from Homo sapiens (1) and Mus musculus (3,4). Promoter set 2 and 3 are both based on a single promoter sequence annotated in only one of the organisms (2,5). The first exon of the corresponding transcript can be mapped on the genomic sequence of the two remaining organisms and is used to determine the location of the corresponding promoter sequences. Each of these promoter sequences is located at the 5' end of an exon annotated for the respective organism and therefore most probably represents a functional regulatory sequence.

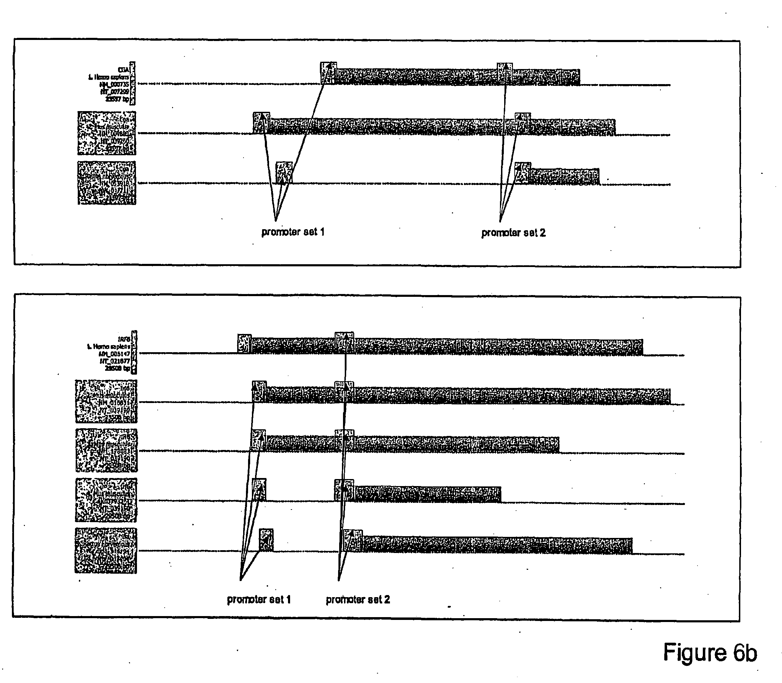

Figure 6b shows the results generated for the two homology groups CGA and IRF6. For the CGA gene one transcript for each of the organisms is available. The transcript annotated for the rat is obviously lacking a 5' leading exon. The originally annotated promoter (assigned to promoter set 2) does not correspond to the promoters annotated tor the two other organisms (assigned to promoter set 1). Due to the additional promoter annotation and the assignment into promoter sets functionally corresponding promoter regions, Le. the members of promoter sets 1 and 2, respectively, from each of the organisms are available tor further analysis.

The results obtained tor the IRF6 locus are comparable. Only the promoter region annotated in the human genome is excluded from the promoter sets 10 indicating either a low degree of conservation between the organisms or an error in the annotation.

The following items are also part of the invention.

The above method wherein the regulatory sequence is selected from the group consisting of promoters, enhancers and repressors, and combinations thereof.

The above method wherein the regulatory sequence is a promoter.

The above method wherein the eukaryotic organisms belong to the same order.

The above method wherein the organisms are mammals.

The above method wherein the transcripts are marked as orthologous when they are pairwise best matches within the respective organisms.

The above method wherein step (a) comprises generating a homology group containing a plurality of orthologous loci in the genome of the respective eukaryotic organisms.

The above method wherein step (b) comprises analysing of transcript sequences from the orthologous loci of a homology group.

The above method wherein two exons are identified as conserved when they have an identical length.

The above method wherein the most 5' located conserved sequence is selected as an anchor.

The above method wherein the location of the target region is defined by the distance between the anchor and the transcriptional start site of a source transcript.

The above method wherein the mapping region in the genome of the first organism has a length of up to about 20,000 bp.

The above method wherein the mapping region in the genome of the first organism has a length of up to about 20% of the distance between the anchor and the transcriptional start site of the source transcript.

The above method wherein the pseudotranscript is generated based on a mapping between the sequence in the target region and the sequence of the first exon of a source transcript, wherein the mapping has to fulfil at least one of the criteria:
(i) the alignment contains ≥ 70% identical nucleotides, ≤ 20% gaps and has ≥ 20bp in length; and
(ii) the score calculated for the alignment normalized by the length of the alignment exceeds a value of 2.

The above method wherein step (d) comprises aligning first exons from transcript or pseudotranscript sequences, assigning transcripts which fulfill predetermined alignment criteria as pair-wise matching partners, and calculating the location of regulatory regions.

The above method wherein calculating is based on the locations of potential transcriptional start sites upstream the first exon sequences.

### References

Needleman SB, Wunsch CD. A general method applicable to the search for similarities in the amino acid sequence of two proteins. J Mol Biol. 1970 Mar,48(3):443-53.
Smith TF, Waterman MS. Identification of common molecular subsequences. J Mol Biol. 1981 Mar 25;147(1):195-7.
Suzuki Y. et al. DBTSS: DataBase of human Transcriptional Start Sites and full-length cDNAs. Nucleic Acids Res. 2002 Jan 1;30(1):328-31.
Suzuki Y. et al. Diverse transcriptional initiation revealed by fine, large-scale mapping of mRNA start sites. EMBO Rep. 2001 May;2(5):388-93.
Waterston RH et al. Mouse Genome Sequencing Consortium. Initial sequencing and comparative analysis of the mouse genome. Nature. 2002 Dec 5;420(6915):520-62.
Okazaki Y. et al. FANTOM Consortium; RIKEN Genome Exploration Research Group Phase I & II Team. Analysis of the mouse transcriptome based on functional annotation of 60,770 full-length cDNAs. Nature. 2002 Dec 5;420(6915):563-73.
Lander ES, et al. International Human Genome Sequencing Consortium. Initial sequencing and analysis of the human genome. Nature. 2001 Feb 15;409(6822):860-921.
Imanishi T. et al. Integrative Annotation of 21,037 Human Genes Validated by Full-Length cDNA Clones. PLoS Biol. 2004 Apr 20.
Ota T. et al. Complete sequencing and characterization of 21,243 full-length human cDNAs. Nat Genet. 2004 Jan;36(1):40-5.

## Claims

1. A method for identifying functionally corresponding regulatory sequences of orthologous transcripts within the genome of eukaroytic organisms which allows to evaluate existing annotation and to transfer high quality annotation from one organism to another where this information is incomplete or missing comprising:
(a) mapping the sequences of a plurality of orthologous transcripts within the genomes of eukaryotic organisms,
(b) identifying at least one sequence which is conserved between the orthologous transcripts of (a), wherein the sequence is a conserved exon,
(c) defining a target region for a conserved sequence in the genome of the respective eukaryotic organism based on the location of the conserved sequence identified in step (b), wherein step (c) comprises selecting a conserved sequence as an anchor for defining the target region,
(d) identifying and/or defining a regulatory sequence within the target region, wherein step (d) comprises generating a pseudotranscript sequence for the target locus, and
(e) optionally assigning the regulatory sequences to groups of functionally corresponding regulatory sequences.

2. The method of claim 1 wherein the regulatory sequence is selected from the group consisting of promoters, enhancers and repressors, and combinations thereof.

3. The method of claim 2 wherein the regulatory sequence is a promoter.

4. The method of claim 1 wherein the eukaryotic organisms belong to the same order.

5. The method of claim 4 wherein the organisms are mammals.

6. The method of claim 1 wherein the transcripts are marked as orthologous when they are pairwise best matches within the respective organisms.

7. The method of claim 1 wherein step (a) comprises generating a homology group containing a plurality of orthologous loci in the genome of the respective eukaryotic organisms.

8. The method of claim 7, wherein step (b) comprises analysing of transcript sequences from the orthologous loci of a homology group.

9. The method of claim 1 wherein two exons are identified as conserved when they have an identical length.

10. The method of claim 1 wherein the most 5' located conserved sequence is selected as an anchor.

11. The method of claim 1 wherein the location of the target region is defined by the distance between the anchor and the transcriptional start site of a source transcript.

12. The method of claim 1 wherein the mapping region in the genome of the first organism has a length of up to about 20,000 bp.

13. The method of claim 1 wherein the mapping region in the genome of the first organism has a length of up to about 20% of the distance between the anchor and the transcriptional start site of the source transcript.

14. The method of claim 1 wherein the pseudotranscript is generated based on a mapping between the sequence in the target region and the sequence of the first exon of a source transcript, wherein the mapping has to fulfil at least one of the criteria:
(i) the alignment contains ≥ 70% identical nucleotides, ≤ 20% gaps and has ≥ 20bp in length; and
(ii) the score calculated for the alignment normalized by the length of the alignment exceeds a value of 2.

15. The method of claim 8 wherein step (d) comprises aligning first exons from transcript or pseudotranscript sequences, assigning transcripts which fulfill predetermined alignment criteria as pair-wise matching partners, and calculating the location of regulatory regions.

16. The method of claim 15 wherein calculating is based on the locations of potential transcriptional start sites upstream the first exon sequences.

17. A computer program product for identifying functionally corresponding regulatory sequences within the genome of eukaryotic organisms which allows to evaluate existing annotation and to transfer high quality annotation from one organism to another where this information is incomplete or missing comprising:
(a) means for mapping the sequences of a plurality of orthologous transcripts within the genomes of a plurality of eukaroytic organisms,
(b) means for identifying at least one sequence which is conserved between the orthologous transcripts of (a), wherein the sequence is a conserved exon,
(c) means for defining a target region for a conserved sequence in the genome of the respective eukaryotic organism based on the location of a conserved sequence identified by means (b), wherein means (c) comprises selecting a conserved sequence as an anchor for defining the target region,
(d) means for identifying and/or defining a regulatory sequence within the target region, wherein means (d) comprises generating a pseudotranscript sequence for the target locus, and
(e) optionally means for assigning the regulatory sequences to groups of functionally corresponding regulatory sequences.

## Patentansprüche

1. Verfahren zum Identifizieren funktional entsprechender regulatorischer Sequenzen orthologer Transkripte innerhalb des Genoms eukaryotischer Organismen, das erlaubt, eine existierende Annotation einzuschätzen und eine Annotation mit hoher Qualität von einem Organismus zu einem anderen zu übertragen, bei dem diese Information unvollständig ist oder fehlt, umfassend:
(a) Abbilden der Sequenzen einer Vielzahl an orthologen Transkripten innerhalb der Genome von eukaryotischen Organismen,
(b) Identifizieren mindestens einer Sequenz, die zwischen den orthologen Transkripten von (a) konserviert ist, wobei die Sequenz ein konserviertes Exon ist,
(c) Definieren einer Zielregion für eine konservierte Sequenz in dem Genom des betreffenden eukaryotischen Organismus, basierend auf dem Ort der konservierten Sequenz, die in Schritt (b) identifiziert wurde, wobei Schritt (c) das Auswählen einer konservierten Sequenz als Anker zum Definieren der Zielregion umfasst,
(d) Identifizieren und/oder Definieren einer regulatorischen Sequenz innerhalb der Zielregion, wobei Schritt (d) das Generieren einer Pseudotranskript-Sequenz für den Zielort umfasst, und
(e) gegebenenfalls Zuordnen der regulatorischen Sequenzen zu Gruppen von funktional entsprechenden regulatorischen Sequenzen.

2. Verfahren nach Anspruch 1, wobei die regulatorische Sequenz aus der Gruppe bestehend aus Promotoren, Enhancern und Repressoren, und Kombinationen davon ausgewählt ist.

3. Verfahren nach Anspruch 2, wobei die regulatorische Sequenz ein Promotor ist.

4. Verfahren nach Anspruch 1, wobei die eukaryotischen Organismen derselben Ordnung angehören.

5. Verfahren nach Anspruch 4, wobei die Organismen Säugetiere sind.

6. Verfahren nach Anspruch 1, wobei die Transkripte als Orthologe **gekennzeichnet** sind, wenn sie paarweise die größte Übereinstimmung innerhalb der jeweiligen Organismen zeigen.

7. Verfahren nach Anspruch 1, wobei Schritt (a) das Generieren einer Homologie-Gruppe umfasst, enthaltend eine Vielzahl an orthologen Orten in dem Genom der jeweiligen eukaryotischen Organismen.

8. Verfahren nach Anspruch 7, wobei Schritt (b) die Analyse von Transkript-Sequenzen der orthologen Orte einer Homologie-Gruppe umfasst.

9. Verfahren nach Anspruch 1, wobei zwei Exons als konserviert bezeichnet werden, wenn sie eine identische Länge haben.

10. Verfahren nach Anspruch 1, wobei die konservierte Sequenz, die am weitesten 5' lokalisiert ist, als ein Anker ausgewählt ist.

11. Verfahren nach Anspruch 1, wobei der Ort der Zielregion durch die Distanz zwischen dem Anker und der Transkriptions-Startstelle eines Ausgangstranskripts definiert ist.

12. Verfahren nach Anspruch 1, wobei die Abbildungsregion in dem Genom des ersten Organismus eine Länge von bis zu ungefähr 20.000 bp hat.

13. Verfahren nach Anspruch 1, wobei die Abbildungsregion in dem Genom des ersten Organismus eine Länge von bis zu ungefähr 20% der Distanz zwischen dem Anker und der Transkriptions-Startstelle des Ausgangstranskripts hat.

14. Verfahren nach Anspruch 1, wobei das Pseudotranskript basierend auf dem Abbilden zwischen der Sequenz in der Zielregion und der Sequenz des ersten Exons eines Ausgangstranskripts generiert wird, wobei das Abbilden mindestens eines der Kriterien:
(i) das Alignment enthält ≥ 70% identische Nukleotide, ≤ 20% Lücken und hat eine Länge von ≥ 20bp; und
(ii) die für das Alignment berechnete, in Beziehung zur Länge des Aligment gesetzte Trefferzahl, übersteigt einen Wert von 2,
erfüllen muss.

15. Verfahren nach Anspruch 8, wobei Schritt (d) das Anordnen erster Exone von Transkript- oder Pseudotranskript-Sequenzen, Zuordnen von Transkripten, die die vorbestimmten Anordnungs-Kriterien als paarweise entsprechende Partner erfüllen, umfassen und Berechnen des Ortes der regulatorischen Regionen.

16. Verfahren nach Anspruch 15, wobei die Berechnung auf den Orten von möglichen Transkriptions-Startstellen stromaufwärts der ersten Exonsequenzen basiert.

17. Produkt in Form eines Computerprogramms zum Identifizieren funktional entsprechender regulatorischer Sequenzen innerhalb des Genoms eukaryotischer Organismen, das erlaubt, existierende Annotationen einzuschätzen und eine Annotation mit hoher Qualität von einem Organismus zu einem anderen zu übertragen, bei dem diese Information unvollständig ist oder fehlt, umfassend:
(a) Mittel zum Abbilden der Sequenzen einer Vielzahl an orthologen Transkripten innerhalb der Genome einer Vielzahl eukaryotischer Organismen,
(b) Mittel zum Identifizieren mindestens einer Sequenz, die zwischen den orthologen Transkripten von (a) konserviert ist, wobei die Sequenz ein konserviertes Exon ist,
(c) Mittel zum Definieren einer Zielregion für eine konservierte Sequenz in dem Genom des betreffenden eukaryotischen Organismus, basierend auf dem Ort der konservierten Sequenz, die durch die Mittel (b) identifiziert wurde, wobei die Mittel (c) das Auswählen einer konservierten Sequenz als Anker zum Definieren der Zielregion umfassen,
(d) Mittel zum Identifizieren und/oder Definieren einer regulatorischen Sequenz innerhalb der Zielregion, wobei die Mittel (d) das Generieren einer Pseudotranskript-Sequenz für den Zielort umfassen, und
(e) gegebenenfalls Mittel zum Zuordnen der regulatorischen Sequenzen zu Gruppen von funktional entsprechenden regulatorischen Sequenzen.

## Revendications

1. Procédé d'identification de séquences régulatrices correspondantes sur le plan fonctionnel de transcripts orthologues dans le génome d'organismes eucaryotes qui permet d'évaluer l'annotation existante et de transférer une annotation de haute qualité d'un organisme à un autre, dans lequel cette information est incomplète ou manquante et comprenant :
(a) la cartographie des séquences d'une pluralité de transcripts orthologues dans les génomes des organismes eucaryotes,
(b) l'identification d'au moins une séquence qui est conservée entre les transcripts orthologues de (a), où la séquence est un exon conservé,
(c) la définition d'une région cible pour une séquence conservée dans le génome de cet organisme eucaryote respectif sur la base de l'emplacement de la séquence conservée identifiée à l'étape (b), où l'étape (c) comprend la sélection d'une séquence conservée comme un ancrage pour définir la région cible,
(d) l'identification et/ou la définition d'une séquence régulatrice dans la région cible, où l'étape (d) comprend la génération d'une séquence de pseudo-transcript pour le locus cible, et
(e) de manière optionnelle l'assignation des séquences régulatrices aux groupes de séquences régulatrices correspondantes sur le plan fonctionnel.

2. Procédé selon la revendication 1, dans lequel la séquence régulatrice est choisie dans le groupe se composant de promoteurs, d'amplificateurs et de répresseurs, et de combinaisons de ceux-ci.

3. Procédé selon la revendication 2, dans lequel la séquence régulatrice est un promoteur.

4. Procédé selon la revendication 1, dans lequel les organismes eucaryotes appartiennent au même ordre.

5. Procédé selon la revendication 4, dans les organismes sont des mammifères.

6. Procédé selon la revendication 1, dans lequel les transcripts sont définis comme orthologues quand ils présentent le meilleur alignement dans leurs organismes respectifs.

7. Procédé selon la revendication 1, dans lequel l'étape (a) comprend la génération d'un groupe d'homologie contenant une pluralité de loci orthologues dans le génome des organismes eucaryotes respectifs.

8. Procédé selon la revendication 7, dans lequel l'étape (b) comprend l'analyse de séquences de transcripts à partir des loci orthologues d'un groupe d'homologie.

9. Procédé selon la revendication 1, dans lequel deux exons sont identifiés comme conservés quand ils sont de longueur identique.

10. Procédé selon la revendication 1, dans lequel la séquence localisée en 5' la plus conservée est choisie comme un ancrage.

11. Procédé selon la revendication 1, dans lequel l'emplacement de la région cible est défini par la distance entre l'ancrage et le site d'initialisation de la transcription d'un transcript source.

12. Procédé selon la revendication 1, dans lequel la région de cartographie dans le génome du premier organisme présente une longueur représentant jusqu'à environ 20 000 pb.

13. Procédé selon la revendication 1, dans lequel la région de cartographie dans le génome du premier organisme présente une longueur représentant jusqu'à environ 20 % de la distance comprise entre l'ancrage et le site d'initialisation de la transcription d'un transcript source.

14. Procédé selon la revendication 1, dans lequel le pseudo-transcript est généré sur la base d'une cartographie entre la séquence dans la région cible et la séquence du premier exon d'un transcript source, dans lequel la cartographie doit remplir au moins un des critères :
(i) l'alignement contient ≥ 70 % de nucléotides identiques, ≤ 20 % de brèche et présente une longueur de ≥ 20 pb ; et
(ii) le score calculé pour l'alignement normalisé par la longueur de l'alignement dépasse une valeur de 2.

15. Procédé selon la revendication 8, dans lequel l'étape (d) comprend l'alignement des premiers exons de séquences de transcript ou de pseudo-transcript, l'assignation des transcripts qui remplissent des critères d'alignement prédéterminés en tant que partenaires d'alignement, et le calcul de l'emplacement des régions régulatrices.

16. Procédé selon la revendication 15, dans lequel le calcul est basé sur les sites d'initialisation de la transcription potentiels situés en amont des premières séquences d'exon.

17. Produit de programme informatique servant à identifier des séquences régulatrices correspondantes sur le plan fonctionnel dans le génome des organismes eucaryotes qui permet d'évaluer l'annotation existante et de transférer une annotation de haute qualité d'un organisme à un autre, dans lequel cette information est incomplète ou manquante et comprenant :
(a) des moyens de cartographier des séquences d'une pluralité de transcripts orthologues dans les génomes d'une pluralité d'organismes eucaryotes,
(b) des moyens d'identifier au moins une séquence qui est conservée entre les transcripts orthologues de (a), où la séquence est un exon conservé,
(c) des moyens de définir une région cible pour une séquence conservée dans le génome de l'organisme eucaryote respectif sur la base de l'emplacement de la séquence conservée identifiée par les moyen (b), dans lequel les moyens (c) comprennent la sélection d'une séquence conservée comme un ancrage pour définir la région cible,
(d) des moyens d'identifier et/ou de définir une séquence régulatrice dans la région cible, où les moyens (d) comprennent la génération d'une séquence de pseudo-transcripts pour le locus cible, et
(e) de manière optionnelle des moyens d'assigner des séquences régulatrices aux groupes de séquences régulatrices correspondantes sur le plan fonctionnel.
